# EUROPEAN PATENT APPLICATION

(11) **EP 2 266 512 A1**
(43) Date of publication of application: **29.12.2010**
(21) Application number: 09726518.5
(22) Date of filing: 31.03.2009
(51) Int. Cl.: A61F 13/15, A61F 13/49, A61F 13/56

(54) **MANUFACTURING METHOD FOR ABSORBENT PRODUCTS AND MANUFACTURING DEVICE FOR ABSORBENT PRODUCTS**

(30) Priority: 31.03.2008 JP 2008094065; 25.03.2009 JP 2009074778
(71) Applicant: Unicharm Corporation, Ehime 799-0111 (JP)
(72) Inventor: The designation of the inventor has not yet been filed
(74) Representative: Eke, Philippa Dianne
(86) International application number: PCT/JP2009/056623
(87) International publication number: WO 2009/123179

(57) **Abstract**

Provided is a manufacturing method for absorbent products that has a process for forming a pair of fold-over portions (50a, 50b), which can be folded over in the direction of conveyance of a flap line and in the direction opposite said conveyance direction, on portions of regions (30A) that constitute a single flap in flap continuum (130) that are continuously conveyed on the flap line, a process wherein fastening members (40a, 40b), which are fastenable to prescribed areas, are placed on the pair of fold-over portions (50a, 50b), and a process wherein the flap continuum (130) are connected with connected front torso-surrounding members or back torso-surrounding members (120a) or (120b) so that each region (30A) that constitutes a single flap, corresponds to areas (20A, 20B), that constitute a single absorbent product in connected front torso-surrounding members or back torso-surrounding members (120a) or (120b).

## Description

### TECHNICAL FIELD

The present invention relates to a manufacturing method and a manufacturing apparatus for absorbent articles each of which includes: a front waistline member, a rear waistline member, and a crotch member that connects the front waistline member and the rear waistline member.

### BACKGROUND ART

In general, in order to be easily put on the wearer, an open-type diaper needs to be provided with: a vertically long absorber (absorber body) that absorbs body fluid from the body of the wearer, a diaper main body to which the absorber is attached, side flaps (hereinbelow referred to as flap) that project outwardly in the width direction of the absorber from the absorber body.

Heretofore, as a manufacturing method for such open-type diaper provided with the flaps, following method has been known (Patent Literature 1, for example).

(1) forming, on continuum of flaps (continuous belt part), a potential cut line between each first belt part sheet fastener and each second belt part sheet fastener both placed in a crossing direction (CD direction) that is orthogonal to the moving direction (MD direction) of the continuum of flaps (flap continuum).

(2) placing the continuum of flaps (continuous belt part) along one edge portion of a continuum of the diaper main bodies (diaper main body continuum), and forming a bonding region in the CD direction.
Patent Literature 1: Japanese Patent Application Publication No. 2006-340862

However, in the above manufacturing method for the open-type diaper, the potential cut line is orthogonal to the MD direction (moving direction) of a flap line on which the continuum of flaps (continuous belt part) is transported. Accordingly, there has been a problem that, when the strength of the potential cut line is increased so as to prevent the continuum of flaps from being torn along the potential cut line on the flap line, a user of the manufactured diaper may find it difficult to tear along the potential cut line.

Further, in the above manufacturing method for the open-type diaper, the potential cut line is orthogonal to the MD direction of the flap line on which the flap continuum (continuous belt part) is transported. Accordingly, when the strength of the potential cut line is formed to be weak so that a user can easily tear, the potential cut line may be torn while the flap continuum is continuously transported on the flap line. Thus, there has been a problem that the flap continuum cannot be stably transported on the flap line, or a manufacturing facility for the diapers becomes complex since high level controls are required for continuously transporting the flap continuum.

The present invention has been made in view of the above-described problems, and has an object of providing a manufacturing method and a manufacturing apparatus for absorbent articles being able to form side flaps that can be easily unfolded and developed by the user of the manufactured diaper, and being able to achieve a continuous transportation of a flap continuum on a flap line without using high level controls.

### DISCLOSURE OF THE INVENTION

A first aspect of the present invention is summarized as a manufacturing method for absorbent articles each including front waistline members, rear waistline members, and crotch members, the crotch members connecting each of the front waistline members and the rear waistline members. The manufacturing method for the absorbent articles according to the present invention includes: placing pairs of folded back portions that can be folded toward a moving direction of a flap line and a direction opposite to the moving direction, in a part of regions each constituting flaps from a flap continuum having a long shape and being continuously transported on the flap line; placing latch members that can be attached to and locked in predetermined regions, on each of the pairs of folded back portions; and bonding a front waistline member continuum or a rear waistline member continuum onto the flap continuum, so that regions each constituting the flaps respectively corresponds to regions each constituting the absorbent articles on the front waistline member continuum or on the rear waistline member continuum.

As described above, the present invention can provide a manufacturing method and a manufacturing apparatus for absorbent articles being able to form side flaps that can be easily unfolded and developed by the user of the manufactured diaper, and to achieve a continuous transportation of the continuum of flaps (continuous belt part) on a flap line without using high level controls.

### BRIEF DESCRIPTION OF DRAWINGS

[Fig. 1] Fig. 1 is a diagram illustrating a manufacturing method for absorbent articles according to a first embodiment of the present invention.
[Fig. 2] Figs. 2A and 2B are diagrams for illustrating a flap bonding step in a manufacturing method for absorbent articles according to the first embodiment of the present invention.
[Fig. 3] Fig. 3 is a diagram illustrating a manufacturing method for absorbent articles according to a modified example 1 of the present invention.
[Fig. 4] Fig. 4 is a diagram illustrating a manufacturing method for absorbent articles according to a modified example 2 of the present invention.
[Fig. 5] Fig. 5 is a diagram illustrating a manufacturing method for absorbent articles according to a modified example 3 of the present invention.
[Fig. 6] Fig. 6 is a diagram illustrating a manufacturing method for absorbent articles according to a modified example 4 of the present invention.
[Fig. 7] Fig. 7 is a diagram illustrating a manufacturing method for absorbent articles according to a modified example 5 of the present invention.
[Fig. 8] Fig. 8 is a diagram illustrating a manufacturing method for absorbent articles according to a modified example 6 of the present invention.
[Fig. 9] Fig. 9 is a diagram illustrating a manufacturing method for absorbent articles according to a modified example 7 of the present invention.
[Fig. 10] Fig. 10 is a perspective view of a latch member placing structure 210 according to a second embodiment of the present invention.
[Fig. 11] Fig. 11 is a perspective view of a folded back portion forming structure 320 according to the second embodiment of the present invention.
[Fig. 12] Fig. 12 is a perspective view of a bonding structure 330 according to the second embodiment of the present invention.

### PREFERRED EMBODIMENTS FOR CARRYING OUT THE INVENTION

### [First Embodiment]

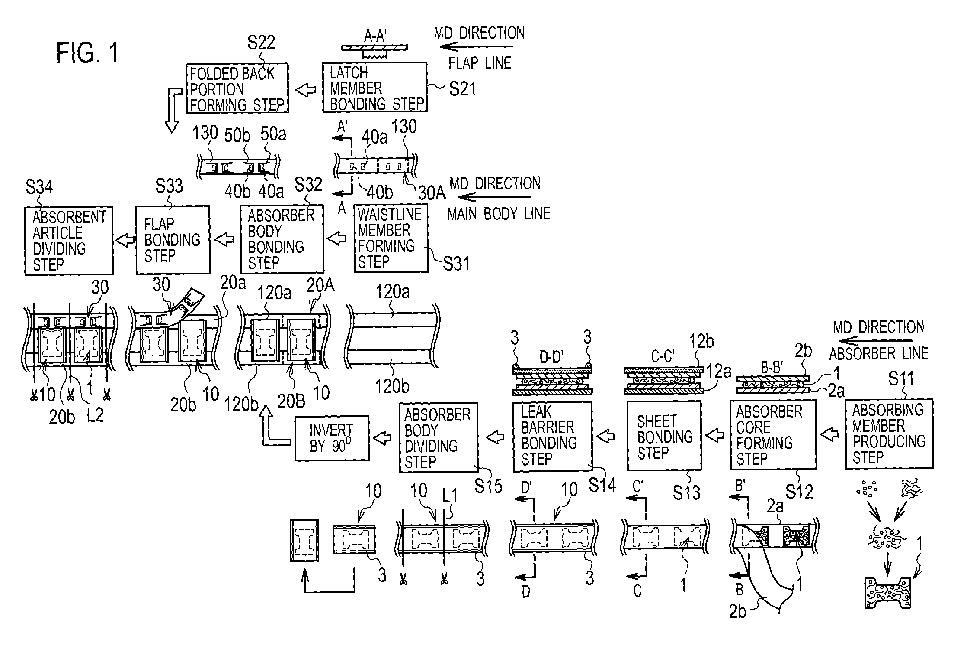

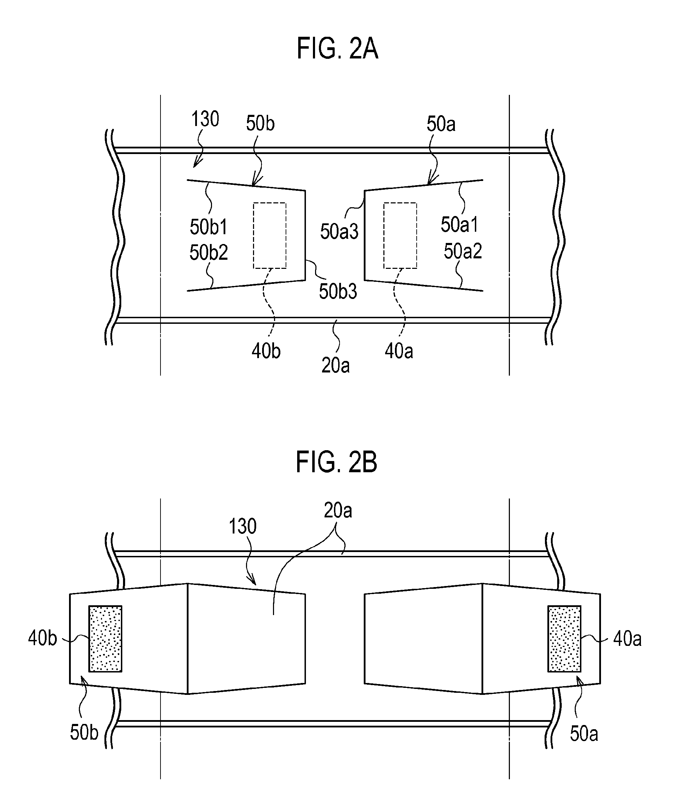

Description will be given of a manufacturing method for absorbent articles according to a first embodiment of the present invention with reference to Figs. 1 and 2.

According to the manufacturing method for absorbent articles according to the first embodiment, absorbent articles, each of which including a front waistline member 20b, a rear waistline member 20a, and a crotch member (absorber body) 10 connecting the front waistline member 20b and the rear waistline member 20a, can be manufactured. For example, open-type diapers can be manufactured.

Hereinafter, with reference to Fig. 1, description will be given of a manufacturing method for absorbent articles according to this embodiment.

It is assumed that, as shown in Fig. 1, the manufacturing method for the absorbent articles according to this embodiment uses: a flap line on which a long continuum of flaps (flap continuum 130) is transported; an absorber line on which a long continuum of crotch members (crotch member continuum 110) is transported; and a main body line on which a pair of a continuum 120b of front waistline members and a continuum 120a of rear waistline members (a pair of a front waistline member continuum 120b and a rear waistline member continuum 120a) is transported.

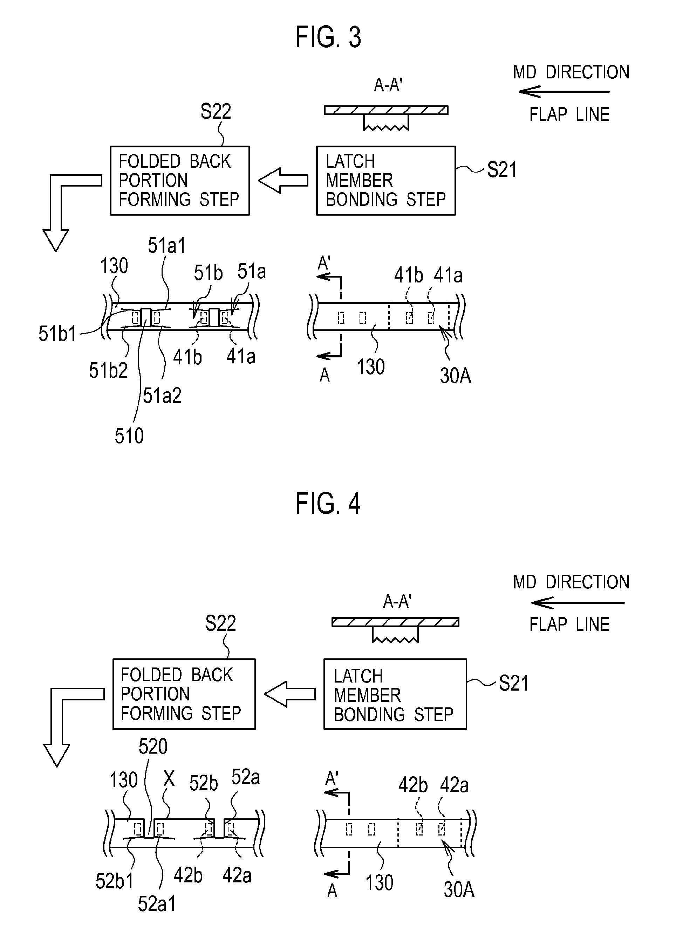

Note that, in this embodiment, an example is described in which an MD direction (moving direction) of the flap line and the MD directions respectively of the main body line and the absorber line are the same, as shown in Fig. 4. However, the present invention is not limited to this example.

Firstly, steps performed on the absorber line, on which the absorbers placed on the crotch members 10 are transported, will be described.

In step S11, a crusher crushes pulp sheets to produce crushed pulps. Thereafter, when the crushed pulps pass through a pulp supply duct, a super absorbent polymer (SAP) is mixed thereto.

Then, the mixture of the crushed pulp and the super absorbent polymer, which is supplied from the pulp supply duct, is layered in a predetermined shape by use of a layer drum, thereby absorber cores 1 (absorbers) are formed.

In step S12, on sheet tissues 2a being continuously transported in the MD direction of the absorber line, the absorber cores 1 are placed by a predetermined interval length in the MD direction of the absorber line.

Subsequently, on the sheet tissues 2a, sheet tissues 2b being continuously transported in the MD direction of the absorber line is layered. Here, on the sheet tissues2a, a plurality of absorber cores 1 is placed along the MD line of the absorber line.

In step S13, top sheets (liquid permeable) 12b being continuously transported in the MD direction of the absorber line are respectively placed on and bonded with the sheet tissues 2b. In addition, back sheets (liquid impermeable) 12a being continuously transported in the MD direction of the absorber line are respectively placed under and bonded with the sheet tissues 2a.

In step S14, leak barriers 3 formed of different members are bonded onto both side edges of the crotch members 10 in the MD direction of the absorber line.

In step S15, the crotch member members (absorber bodies10) being continuously transported in the MD direction of the absorber line are transported by use of a rotation of a rotating drum. Concurrently, the crotch member continuum 110 is cut in the CD direction by a predetermined interval length, by use of a cutter roll arranged opposite to the outer peripheral surface of the rotating drum. Thereby the crotch members 10 are formed.

Secondly, steps performed on the flap line, on which the flaps 30 to be placed on the rear waistline members 20a are transported, will be described.

In step S21, in a part of regions 30A each constituting one flap on the flap continuum 130 having a long shape and being continuously transported on the flap line, latch members 40a and 40b that can be attached to and locked in predetermined regions are placed.

Specifically, on the flap continuum 130, the latch members 40a and 40b are bonded to the flap continuum 130, by a predetermined interval length. Here, a length of each of the latch members 40a and 40b is set to be shorter than the flap continuum 130 in a CD direction of the flap line.

In this regard, when hook members (male members) are placed as the latch members 40a and 40b, hooked members (female members) are provided as latched members in the predetermined regions of the front waistline members 20b.

Note that, in the present embodiment, the front waistline members 20b are made of a non-woven fabric. Accordingly, the predetermined regions of the front waistline members 20b themselves can serve as the hooked members even when the hooked members are not additionally provided.

In step S22, in the regions including the latch members 40a and 40b in the part of the aforementioned regions 30A, pairs of folded back portions 50a and 50b that can be folded back to the MD direction (moving direction) of the flap line and a direction opposite to the MD direction are formed.

As shown in Fig. 2A, each of the folded back portions 50a includes: a first cut portion (first slit) 50a3 formed to extend in the CD direction of the flap line (that is, the direction crossing the moving direction), and two second cut portions (second slits) 50a1 and 50a2 formed to extend in the MD direction of the flap line.

Meanwhile, each of the folded back portions 50b includes: a first cut portion (first slit) 50b3 formed to extend in the CD direction of the flap line, and two second cut portions (second slits) 50b1 and 50b2 formed to extend in the MD direction of the flap line.

Here, consider the case where each of the folded back portions 50b is folded back outwardly in the MD direction of the flap line, and the folded back portion 50a is folded back outwardly in the opposite MD direction of the flap, line like hinges as shown in Fig. 2B. Even in this case, the flap continuum 130 is not entirely folded back in the CD direction of the flap line until each of the absorbent article is cut. Accordingly, it is possible to prevent the flap continuum 130 from being turned over while being transported.

In this regard, at least a part of the first cut portion (first slit) 50a3 and 50b3, and the second cut portions (second slits) 50a1, 50a2, 50b1, and 50b2 may be formed by a potential cut line, such as a perforated line.

In particular, it is preferable that a part of the first cut portions (first slit) 50a3 and 50b3 be formed by the potential cut line such as the perforated line. This configuration makes it possible to prevent the flap continuum 130 from being turned over while the flap continuum 130 is transported on the flap line.

Further, a part of the first cut portion (first slit) 50a3 and 50b3, and the second cut portions (second slits) 50a1, 50a2, 50b1, and 50b2 may be straight or curved.

Furthermore, steps S21 and S22 described above may be performed in reverse order. To be more specific, in step S22, the latch members 40a and 40b that can be attached to and locked in the predetermined regions may be placed in each of the folded back portions 50a and 50b respectively, after the pairs of folded back portions 50a and 50b that can be folded back to the MD direction of the flap line and a direction opposite to the MD direction are formed in the part of regions 30A each constituting one flap 30 on the flap continuum 130 having a long shape and being continuously transported on the flap line.

Thirdly, steps performed on the main body line, on which the front waistline member continuum 120b and the rear waistline member continuum 120a are transported, will be described.

In step S31, a pair of continuums of waistline members (the front waistline member continuum 120b and the rear waistline member continuum 120a) both having a long shape is produced from non-woven fabric and is transported in the MD direction of the main body line.

Note that, the front waistline member continuum 120b and the rear waistline member continuum 120a may have stretching properties.

In step S32, the crotch members 10 each formed in step S15 on the absorber line is placed while being spaced apart from one another in the MD direction of the main body line, between the front waistline member continuum120b and the rear waistline member continuum 120a. Here, both of the front waistline member continuum120b and the rear waistline member continuum 120a have a long shape and are continuously transported on the main body line.

In step S33, on the main body line, the flap continuum 130 is bonded to the rear waistline member continuum 120a so that the regions 30A each constituting the flaps 30 on the flap continuum 130 respectively corresponds to the regions 20A each constituting the absorbent articles (rear waistline members 20a).

In other words, the flap continuum 130 is bonded to the rear waistline member continuum 120a so that the latch members 40a and 40b can be placed in the vicinity of the center of the rear waistline members 20a of the absorbent articles.

Specifically, it is preferable that the regions 30A and 20A are bonded along edge portions in the width direction of the absorbent articles.

In this regard, in step S33, it is preferable that the flap continuum 130 be bonded on the rear waistline member continuum 120a by use of a thermo compression or the like, using an embossing roller or ultrasonic waves. Further, the thermoplastic resin may be applied onto the entire back surface side of the flap continuum 130. Furthermore, the thermoplastic resin may be applied onto only a part of the back surface side of the flap continuum 130.

Moreover, in step S33, the latch members 40a and 40b may be placed on the pair of folded back portions 50a and 50b respectively, and may be attached to and locked on the rear waistline member continuum 120a.

In step S34, on the main body line, the front waistline member continuum 120b and the rear waistline member continuum 120a are cut in the CD direction of the main body line by a predetermined interval length.

Specifically, the front waistline member continuum 120b and the rear waistline member continuum 120b are cut so that each lengths L3 of the front waistline members 20b in the width direction of the absorbent articles and each lengths L2 of the rear waistline members 20a in the width direction of the absorbent articles can be equal.

Note that, in the above-described example, description has been given of the example in which the flap continuum 130, on which the latch members 40a and 40b that can be attached to and locked in the predetermined regions of the front waistline members 20b are placed, is placed on the rear waistline member continuum 120a. However, the present invention is not limited to this example but can be applied to an example in which the flap continuum 130, on which the latch members 40a and 40b that can be attached to and locked in the predetermined regions of the front waistline members 20b are placed, is placed on the front waistline member continuum 120b.

According to the manufacturing method for the absorbent articles according to the first embodiment of the present invention, the pairs of folded back portions 50a and 50b are formed so that the flap continuum 130 is not entirely folded back in the CD direction of the flap line until each of the absorbent article is cut. Accordingly, it is possible to form the absorbent articles that can be easily unfolded and developed by the user, and to prevent a problem that the absorbent articles are torn off on the flap line and thereby the flap continuum cannot be continuously transported on the flap line, or a problem that high level controls are required for continuously transporting the flap continuum on the flap line and thereby the manufacturing facility for the diapers becomes complex.

According to the manufacturing method for the absorbent articles of the first embodiment of the present invention, not the flaps 30, the flap continuum 130 is placed on the rear waistline continuum 120a. Accordingly, sophisticated manufacturing facility is not required.

According to the manufacturing method for the absorbent articles of the first embodiment of the present invention, the front waistline continuum 120b and the rear waistline continuum 120a are continuously transported and thereby each end portion of the front waistline continuum 120b and the rear waistline continuum 120a can be easily stacked, and the front waistline member continuum 120b and the rear waistline member continuum 120a thus stacked can be simultaneously cut. Thus, open-type diapers and pants-type diapers can be manufactured by the same cutting step. Therefore, the open-type diaper and the pants-type diaper can be manufactured without changing the cutting step or a cutter itself.

### (Modified Example 1)

With reference to Fig. 3, description will be given of a manufacturing method for absorbent articles according to a modified example 1 of the present invention. Hereinafter, the manufacturing method for the absorbent articles according to the modified example 1 of the present invention will be described, mainly focusing on the differences from the manufacturing method for the absorbent articles according to the first embodiment of the present invention.

As shown in Fig. 3, on the process performed on the flap line, in step S22, latch members 41a and 41b that can be attached to and locked in the predetermined regions are placed, in the part of regions 30A each constituting one flap 30 on the flap continuum 130 having a long shape and being transported on the flap line.

In step S22, in each regions including the latch members 41a and 41b in a part of regions 30A, each pairs of folded back portions 51a and 51b that can be folded back to the MD direction of the flap line and a direction opposite to the MD direction is formed.

As shown in Fig. 3, it is assumed that each pairs of folded back portions 51a and 51b includes: a first cut-out portion (first trimming) 510 formed to extend in the CD direction of the flap line, and four second cut portions (second slits) 51a1, 51a2, 51b1 and 51b2 each formed to extend in the MD direction of the flap line.

Here, the first cut-out portion (first trimming) 510 is placed approximately in the vicinity of the center region in the CD direction of the flap line, as shown in Fig. 3.

In this case, it is preferable that at least a part of the second cut portions (second slits) 51a1, 51a2, 51b1 and 51b2 be formed by the potential cut line such as the perforated line. This configuration makes it possible to prevent the folded back portions 51a and 51b from being turned over while the flap continuum 130 is transported on the flap line.

### (Modified Example 2)

With reference to Fig. 4, description will be given of a manufacturing method for absorbent articles according to a modified example 2 of the present invention. Hereinafter, the manufacturing method for the absorbent articles according to the modified example 2 of the present invention will be described, mainly focusing on the differences from the manufacturing method for the absorbent articles according to the first embodiment of the present invention.

As shown in Fig. 4, on the process performed on the flap line, in step S22, latch members 42a and 42b that can be attached to and locked in the predetermined regions are placed, in the part of regions 30A each constituting one flap 30 on the flap continuum 130 having a long shape and being transported on the flap line.

In step S22, in each regions including the latch members 42a and 42b in a part of regions 30A, each pairs of folded back portions 52a and 52b that can be folded back to the MD direction of the flap line and a direction opposite to the MD direction is formed.

As shown in Fig. 4, it is assumed that each pairs of folded back portions 52a and 52b includes: a first cut-out portion (first trimming) 520 formed to extend in the CD direction of the flap line, and two second cut portions (second slits) 52a1 and 52b1 each formed to extend in the MD direction of the flap line.

Here, as shown in Fig. 4, the first cut-out portion (first trimming) 520 is placed so as to extend in the CD direction from the center of the flap line to the edge X.

In this case, it is preferable that at least a part of the second cut portions (second slits) 51a1 and 52b1 be formed by the potential cut line such as the perforated line. This configuration makes it possible to prevent the folded back portions 52a and 52b from being turned over while the flap continuum 130 is transported on the flap line.

### (Modified Example 3)

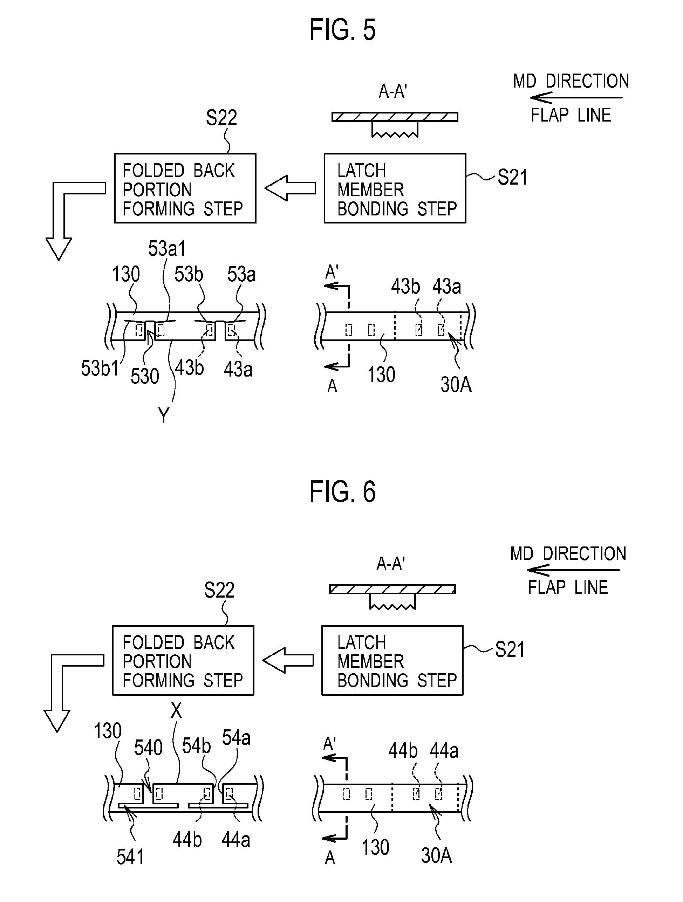

With reference to Fig. 5, description will be given of a manufacturing method for absorbent articles according to a modified example 3 of the present invention. Hereinafter, the manufacturing method for the absorbent articles according to the modified example 3 of the present invention will be described, mainly focusing on the differences from the manufacturing method for the absorbent articles according to the first embodiment of the present invention.

As shown in Fig. 5, on the process performed on the flap line, in step S21, in the part of regions 30A each constituting one flap 30 on the flap continuum 130 having a long shape and being transported on the flap line, latch members 43a and 43b that can be attached to and locked in the predetermined regions are placed.

In step S22, in each regions including the latch members 43a and 43b in a part of regions 30A, each pairs of folded back portions 53a and 53b that can be folded back to the MD direction of the flap line and a direction opposite to the MD direction is formed.

As shown in Fig. 5, it is assumed that each pairs of folded back portions 53a and 53b includes: a first cut-out portion (first trimming) 530 formed to extend in the CD direction of the flap line, and two second cut portions (second slits) 53a1 and 53b1 each formed to extend in the MD direction of the flap line.

Here, as shown in Fig. 5, the first cut-out portion (first trimming) 530 is placed so as to extend in the CD direction from the center of the flap line to the edge Y (the edge opposite to the aforementioned edge X).

In this case, it is preferable that at least a part of the second cut portions (second slits) 53a1 and 53b1 be formed by the potential cut line such as the perforated line. This configuration makes it possible to prevent the folded back portions 53a and 53b from being turned over while the flap continuum 130 is transported on the flap line.

### (Modified Example 4)

With reference to Fig. 6, description will be given of a manufacturing method for absorbent articles according to a modified example 4 of the present invention. Hereinafter, the manufacturing method for absorbent articles according to the modified example 4 of the present invention will be described, mainly focusing on the differences from the manufacturing method for absorbent articles according to the first embodiment of the present invention.

As shown in Fig. 6, on the process performed on the flap line, in step S21, in the part of regions 30A each constituting one flap 30 on the flap continuum 130 having a long shape and being transported on the flap line, latch members 44a and 44b that can be attached to and locked in the predetermined regions are placed.

In step S22, in each regions including the latch members 44a and 44b in a part of regions 30A, each pairs of folded back portions 54a and 54b that can be folded back to the MD direction of the flap line and a direction opposite to the MD direction is formed.

As shown in Fig. 6, it is assumed that each pairs of folded back portions 54a and 54b includes: a first cut-out portion (first trimming) 540 formed to extend in the CD direction of the flap line, and a second cut-out portion (second trimming) 541 formed to extend in the MD direction of the flap line.

Here, as shown in Fig.6, the first cut-out portion (first trimming) 540 is placed so as to extend in the CD direction from the center of the flap line to the edge X.

### (Modified Example 5)

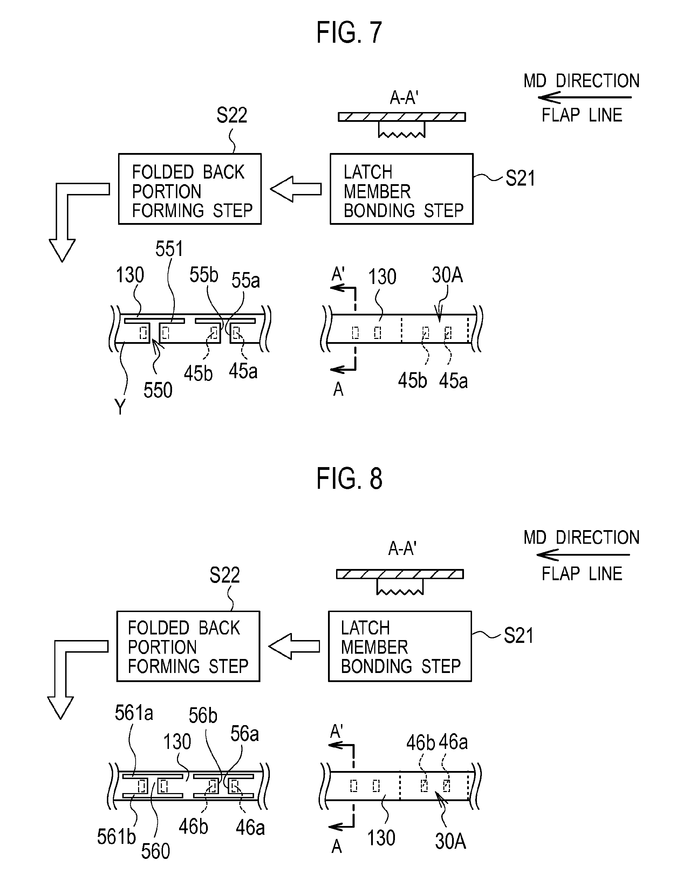

With reference to Fig. 7, description will be given of a manufacturing method for absorbent articles according to a modified example 1 of the present invention. Hereinafter, the manufacturing method for absorbent articles according to the modified example 5 of the present invention will be described, mainly focusing on the differences from the manufacturing method for absorbent articles according to the first embodiment of the present invention.

As shown in Fig. 7, on the process performed on the flap line, in step S21, in the part of regions 30A each constituting one flap 30 on the flap continuum 130 having a long shape and being transported on the flap line, latch members 45a and 45b that can be attached to and locked in the predetermined regions are placed.

In step S22, in each regions including the latch members 45a and 45b in a part of regions 30A, each pairs of folded back portions 55a and 55b that can be folded back to the MD direction of the flap line and a direction opposite to the MD direction is formed.

As shown in Fig. 7, it is assumed that each pairs of folded back portions 55a and 55b includes: a first cut-out portion (first trimming) 550 formed to extend in the CD direction of the flap line, and a second cut-out portion (second trimming) 551 formed to extend in the MD direction of the flap line.

Here, as shown in Fig.7, the first cut-out portion (first trimming) 550 is placed so as to extend in the CD direction from the center of the flap line to the edge Y (the edge opposite to the aforementioned edge X).

### (Modified Example 6)

With reference to Fig. 8, description will be given of a manufacturing method for absorbent articles according to a modified example 6 of the present invention. Hereinafter, the manufacturing method for an absorbent article according to the modified example 6 of the present invention will be described, mainly focusing on the differences from the manufacturing method for the absorbent articles according to the first embodiment of the present invention.

As shown in Fig. 8, on the process performed on the flap line, in step S21, in the part of regions 30A each constituting one flap 30 on the flap continuum 130 having a long shape and being transported on the flap line, latch members 46a and 46b that can be attached to and locked in the predetermined regions are placed.

In step S22, in each regions including the latch members 46a and 46b in a part of regions 30A, each pairs of folded back portions 56a and 56b that can be folded back to the MD direction of the flap line and a direction opposite to the MD direction is formed.

As shown in Fig. 8, it is assumed that the pairs of folded back portions 56a and 56b each includes: a first cut-out portion (first trimming) 560 formed to extend in the CD direction of the flap line, and two second cut-out portions (second trimmings) 561a and 561b each formed to extend in the MD direction of the flap line.

Here, as shown in Fig.8, the first cut-out portion (first trimming) 560 is placed so as to extend in the CD direction from the second cut-out portion 561a to the second cut-out portion 561b.

### (Modified Example 7)

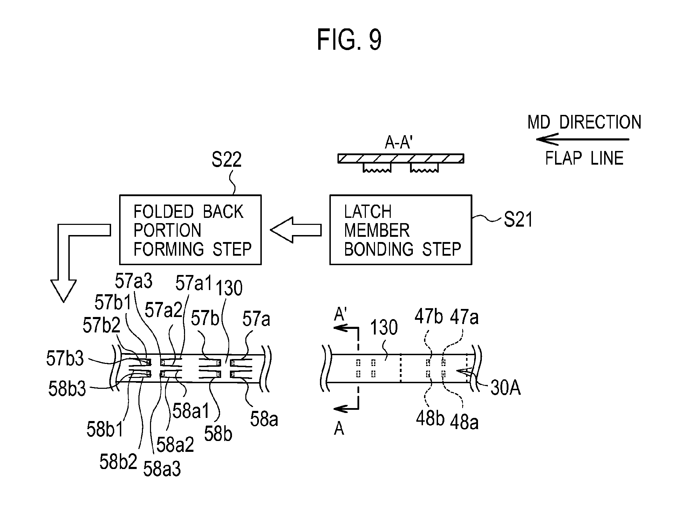

With reference to Fig. 9, description will be given of a manufacturing method for absorbent articles according to a modified example 7 of the present invention. Hereinafter, the manufacturing method for absorbent articles according to the modified example 7 of the present invention will be described, mainly focusing on the differences from the manufacturing method for absorbent articles according to the first embodiment of the present invention.

As shown in Fig. 9, on the process performed on the flap line, in step S21, in the part regions 30A each constituting one flap 30 on the flap continuum 130 having a long shape and being transported on the flap line, four latch members 47a, 47b, 48a and 48b that can be attached to and locked in the predetermined regions are placed.

In step S22, in each regions including the latch members 47a and 47b in a part of regions 30A, pairs of folded back portions 57a and 57b that can be folded back to the MD direction of the flap line and a direction opposite to the MD direction are formed.

As shown in Fig. 9, it is assumed that the folded back portion 57a includes: a first cut portion (first slit) 57a3 formed to extend in the CD direction of the flap line, and two second cut portions (second slits) 57a1 and 57a2 each formed to extend in the MD direction of the flap line.

In addition, it is assumed that the folded back portion 58a includes: a first cut portion (first slit) 58a3 formed to extend in the CD direction of the flap line, and two second cut portions (second slits) 58a1 and 58a2 each formed to extend in the MD direction of the flap line.

Further, it is assumed that the folded back portion 57b includes: a first cut portion (first slit) 57b3 formed to extend in the CD direction of the flap line, and two second cut portions (second slits) 57b1 and 57b2 each formed to extend in the MD direction of the flap line.

Moreover, it is assumed that the folded back portion 58b includes: a first cut portion (first slit) 58b3 formed to extend in the CD direction of the flap line, and two second cut portions (second slits) 58b1 and 58b2 each formed to extend in the MD direction of the flap line.

In this regard, in step S21, four latch members may be placed in a part of regions 30A each constituting one flap 30 on the flap continuum 130 having a long shape and being transported on the flap line. Further, in step S22, more than two folded back portions may be formed in the region including these latch members.

In this case, it is preferable that at least a part of the first cut portions (first slits) 57a3, 57b3, 58a3, and 58b3 be formed by the potential cut line such as the perforated line. This configuration makes it possible to prevent the folded back portions 57a, 57b, 58a and 58b from being turned over while the flap continuum 130 is transported on the flap line.

### [Second Embodiment]

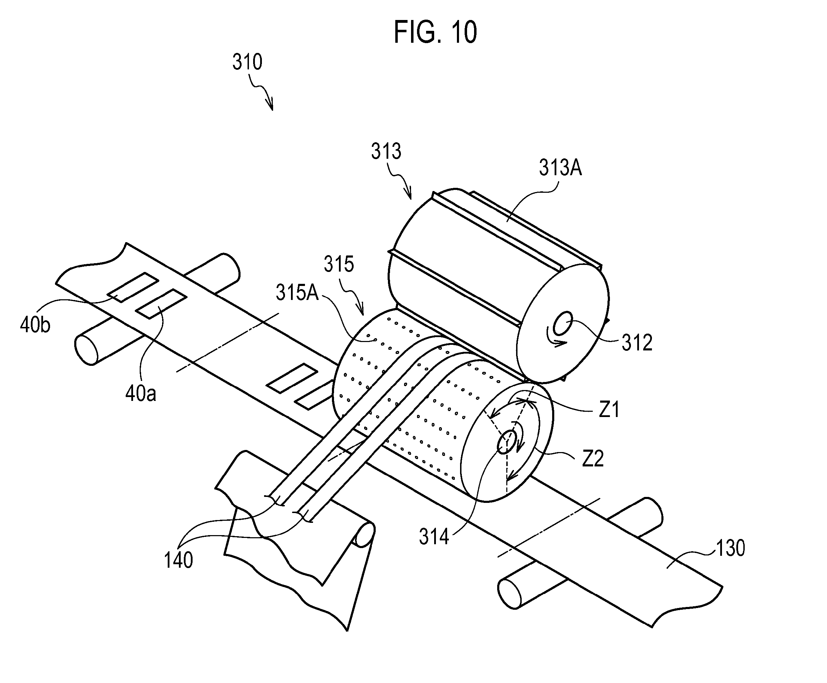

Hereinbelow, a manufacturing apparatus for absorbent articles according to the second embodiment will be described by referring to Figs. 10 to 12. The manufacturing apparatus for the absorbent articles according to the second embodiment is provided with: a latch member placing structure 310; a folded back portion forming structure 320; and a bonding structure 330.

First, the latch member placing structure 310 according to the second embodiment will be described by referring to the accompanying drawings. Fig. 10 is a perspective view of the latch member placing structure 310 according to the second embodiment of the present invention.

As shown in Fig. 10, the latch member placing structure 310 places the latch members 40a and 40b that can be attached to and locked in a predetermined region, on the flap continuum 130 having a long shape and being continuously transported on the flap line (above-described step S21).

The latch member placing structure 310 includes: an upper blade roll 313 configured to rotate by using a shaft 312 as a center; and a lower blade roll 315 configured to rotate by using a shaft 314 as a center.

The upper blade roll 313 is provided with a plurality of blades 313A placed by a predetermined interval length on the outer peripheral surface thereof. Here, the plurality of blades 313A is placed along the shaft center 312. The lower blade roll 315 is provided with: a suction hole 315A configured to suck the flaps 30 formed by cutting the flap continuum 130; and a stationary blade (not shown) configured to cut the flap continuum 130 with the blade 313A.

On the peripheral surface of the lower blade roll 315 includes a first zone Z1 that sucks and holds the flap continuum 130 with a predetermined suction force, and a second zone Z2 that sucks and holds the flaps 30 formed by cutting the flap continuum 130, with a suction force stronger than that of the first zone Z1.

When the latch member continuum 140 passes through the first zone Z1, while slipping around the peripheral surface of the lower blade roll 315, the latch member continuum 140 is sucked by the suction holes 315A formed on the first zone Z1 so as to be held by the peripheral surface of the lower blade roll 315. At this time, the latch member continuum 140 is cut between the blade 313A and the stationary blade. Thereby, the latch members 40a and 40b are formed.

When the latch members 40a and 40b pass through the second zone Z2, without slipping around the peripheral surface of the lower blade roll 315, the flaps 30 are sucked by the suction holes 315A formed on the second zone Z2 so as to be held by the peripheral surface of the lower blade roll 315. Then, the latch members 40a and 40b approach the rear front waistline member continuum 120a by the rotation of the lower blade roll 315 so as to be attached thereon.

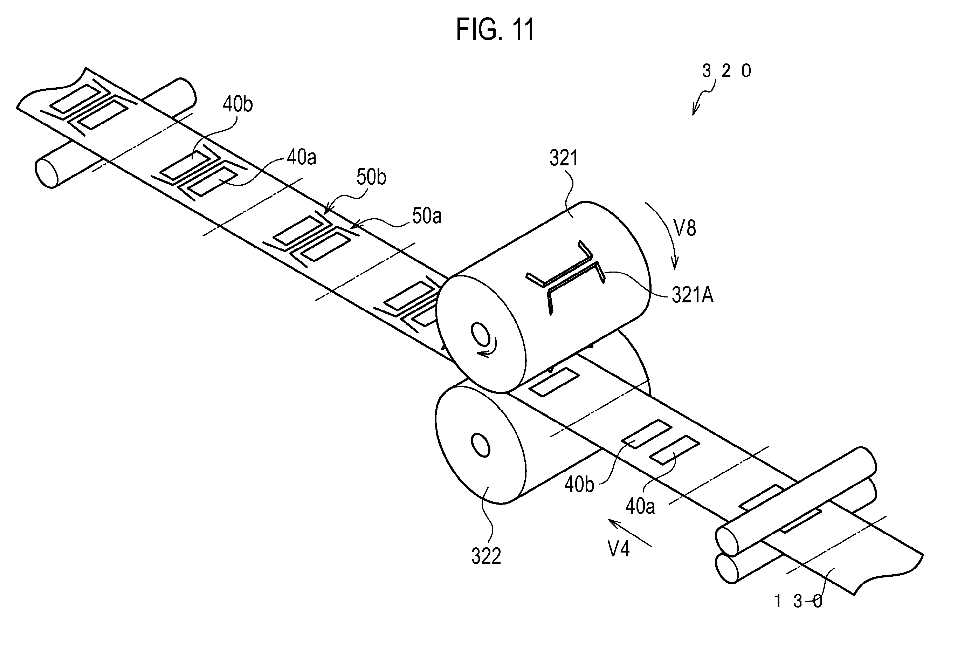

Next, the folded back portion forming structure 320 according to the second embodiment of the present invention will be described by referring to the accompanying drawings. Fig. 11 is a perspective view of the folded back portion forming structure 320 according to the second embodiment of the present invention.

As shown in Fig. 11, the folded back portion forming structure 320 forms pairs of folded back portions 50a and 50b that can be folded back to the MD direction and to the direction opposite to the MD direction.

The folded back portion forming structure 320 is provided with: a cutting roll 321 configured to rotate by using a shaft (not shown) as a center; and a nipping roll 322 configured to nip the flap continuum 130 between the cutting roll 231 and the nipping roll 232.

The circumferential velocity (V₇) of the cutting roll 321 is substantially same as the moving velocity (V₄) of the flap continuum 130. The cutting roll 231 includes a folding back process blade 321A for forming the folding portions 50a and 50b. The folding back process blade 321A is formed in corresponding to the shape of the folding portions 50a and 50b.

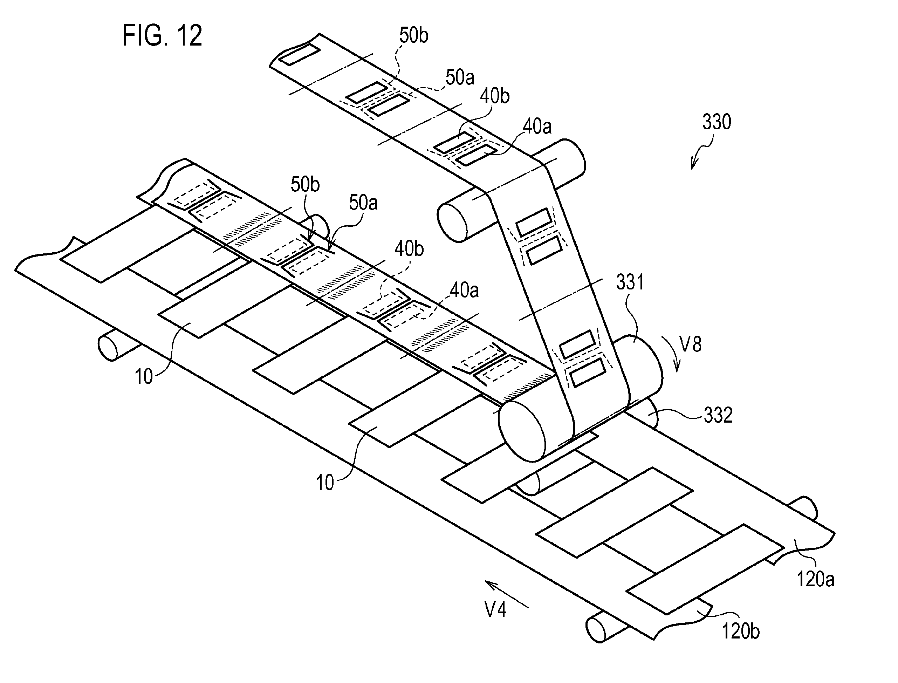

Subsequently, the bonding structure 330 according to the second embodiment of the present invention will be described by referring to the accompanying drawings. Fig. 12 is a perspective view of the bonding structure 330 according to the second embodiment of the present invention.

As shown in Fig. 12, the boding structure 330 bonds the rear waistline member continuum 120a and the flaps 30, so that each of the area constituting each flaps 30 corresponds to each area constituting each of the absorbent article on the rear waistline member continuum 120a.

The bonding structure 330 bonds the flap continuum 130 and the rear waistline member continuum 120a on the main body line (above-described step S33), so that the MD direction of the flap line corresponds to the MD direction of the main body line. The bonding structure 330 is provided with a pair of rollers 331 and 332. Note than, the circumferential velocity (V₈) of the pair of rollers 331 and 332 is substantially same as the moving velocity (V₄) of the flap continuum 130.

The roller 331 guides the flap continuum 130 to the rear waistline member continuum 120a. Meanwhile, the roller 332 nips the rear waistline member continuum 120a and the flap continuum 130, and faces the roller 331. The pair of rollers 331 and 332 nips the rear waistline member continuum 120a and the flap continuum 130 therebetween, thereby the bonding structure 330 bonds the rear waistline member continuum 120a and the flaps 30.

### [Other Embodiments]

It is a matter of course that orders of performing the steps in the above-described manufacturing method for the absorbent articles is not limited to those described above as long as the absorbent article can be manufactured, and the steps to be performed can be appropriately selected in accordance with the intended purpose.

As described above, the present invention has been described in detail by using the above-described embodiments. However, it is obvious for a person skilled in the art that the present invention is not limited to the embodiments described in this specification. The present invention can be implemented as a modification and an amended embodiment without departing from the content and scope of the present invention which is defined by the description of the scope of claims. Accordingly, the description of the present invention is intended to give description as an example and does not have any meaning to limit the present invention.

This application is based upon and claims the benefit of priority from the prior Japanese Patent Application No. JP 2008-094065, filed on March 31, 2008, and Japanese Patent Application No. JP 2009-074778, filed on March 25, 2009, the entire contents of which are incorporated herein by reference.

### INDUSTRIAL APPLICABILITY

As described above, according to absorbent articles of the present invention, there can be provided a manufacturing method for absorbent articles being able to form side flaps that can be easily unfolded and developed by the user of the manufactured diaper, and being able to achieve a continuous transportation of a flap continuum on a flap line without using high level controls.

## Claims

1. A manufacturing method for absorbent articles each including front waistline members, rear waistline members, and crotch members, the crotch members connecting each of the front waistline members and the rear waistline members, comprising:
placing pairs of folded back portions that can be folded toward a moving direction of a flap line and a direction opposite to the moving direction, in a part of regions each constituting flaps from a flap continuum having a long shape and being continuously transported on the flap line;
placing latch members that can be attached to and locked in predetermined regions, on each of the pairs of folded back portions; and
bonding a front waistline member continuum or a rear waistline member continuum onto the flap continuum, so that regions each constituting the flaps corresponds to regions each constituting the absorbent articles on the front waistline member continuum or on the rear waistline member continuum.

2. The manufacturing method for the absorbent articles according to claim 1, wherein, in placing the pairs of folded back portions, each of the pairs of folded back portions include: at least any one of a first cut portion and a first cut-out portion both formed to extend in a direction crossing the moving direction of the flap line; and at least any one of a second cut portion and a second cut-out portion both formed to extend in the moving direction of the flap line.

3. The manufacturing method for the absorbent articles according to claim 1, wherein,
in the bonding, the latch members placed on the pairs of folded back portions are attached to and locked on the front waistline member continuum or the rear waistline member continuum.

4. The manufacturing method for the absorbent articles according to claim 1, further comprising:
placing the crotch members so that each of the crotch members is apart from each other in a moving direction of a main body line, between the front waistline member continuum and the rear waistline member continuum both having a long shape and being transported continuously on the main body line.

5. The manufacturing method for the absorbent articles according to claim 1, wherein,
in the bonding, the front waistline member continuum or the rear waistline member continuum is bonded onto the flap continuum.

6. The manufacturing method for the absorbent articles according to claim 1, wherein,
in the bonding, the flap continuum is placed so that each of the flaps covers edge portions of the absorbent articles placed under the crotch members.

7. The manufacturing method for the absorbent articles according to claim 1, further comprising:
cutting the front waistline member continuum and the rear waistline member continuum so that each lengths of the front waistline members in a width direction of the absorbent articles is equal to each length of the rear waistline members in a width direction of the absorbent articles.

8. A manufacturing apparatus for absorbent articles each including front waistline members, rear waistline members, and crotch members, the crotch members connecting each of the front waistline members and each of the rear waistline members, comprising:
a latch member placing structure configured to place latch members that can be attached to and locked in predetermined regions, on a flap continuum having a long shape and being continuously transported on a flap line;
a folded back portion forming structure configured to form pairs of folded back portions that can be folded toward a moving direction of the flap line and a direction opposite to the moving direction, in a part of regions each constituting flaps from the flap continuum; and
a bonding structure configured to bond a front waistline member continuum or a rear waistline member continuum onto the flap continuum, so that regions each constituting the flaps respectively corresponds to regions each constituting the absorbent articles on the front waistline member continuum or on the rear waistline member continuum.
